# EUROPEAN PATENT SPECIFICATION

(11) **EP 3 901 170 B1**
(45) Date of publication and mention of the grant of the patent: **06.05.2026**
(21) Application number: 19900628.9
(22) Date of filing: 01.10.2019
(51) Int. Cl.: C07K 16/26, C12N 15/09, C12N 15/02, A61K 39/395, G01N 33/577, C07K 14/575

(54) **RECOMBINANT ANTIBODY OF ANTI-HUMAN N-TERMINAL BRAIN NATRIURETIC PEPTIDE PRECURSOR**
REKOMBINANTER ANTIKÖRPER VON VORLÄUFER DES ANTI-HUMANEM N-TERMINALEN NATRIURETISCHEN PEPTIDS
ANTICORPS DE RECOMBINAISON DIRIGÉ CONTRE LE PRÉCURSEUR PEPTIDIQUE NATRIURÉTIQUE CÉRÉBRAL N-TERMINAL HUMAIN

(30) Priority: 19.12.2018 CN 201811557468
(43) Date of publication of application: 27.10.2021
(73) Proprietor: Fapon Biotech Inc., Shenzhen, Guangdong 518000 (CN)
(72) Inventor: MENG, Yuan, Dongguan, Guangdong 523000 (CN); ZHONG, Dongmei, Dongguan, Guangdong 523000 (CN); YE, Qingni, Dongguan, Guangdong 523000 (CN); LIANG, Bi, Dongguan, Guangdong 523000 (CN); YOU, Hui, Dongguan, Guangdong 523000 (CN); MA, Qiuyan, Dongguan, Guangdong 523000 (CN); LI, Weizhi, Dongguan, Guangdong 523000 (CN)
(74) Representative: Novagraaf Group
(86) International application number: PCT/CN2019/109792
(87) International publication number: WO 2020/125136

(56) References cited:
- EP-A1- 3 360 570
- WO-A1-2009/066010
- WO-A2-2007/138163
- CN-A- 103 087 191
- CN-A- 106 916 225
- WILKINS MICHAEL D. ET AL: "Quantum dot enabled lateral flow immunoassay for detection of cardiac biomarker NT-proBNP", SENSING AND BIO-SENSING RESEARCH, vol. 21, 1 November 2018 (2018-11-01), pages 46 - 53, XP055897398, ISSN: 2214-1804, DOI: 10.1016/j.sbsr.2018.10.002
- KIM HYUNG-YONG ET AL: "Affinity Maturation of Monoclonal Antibodies by Multi-Site-Directed Mutagenesis", 1 January 2014, MONOCLONAL ANTIBODIES : METHODS AND PROTOCOLS / ED. BY VINCENT OSSIPOW; NICOLAS FISCHER; [METHODS IN MOLECULAR BIOLOGY , ISSN 1064-3745], NEW YORK [U.A.] : HUMANA PR., 2014, US, PAGE(S) 407 - 420, ISBN: 978-1-62703-991-8, XP008182231
- NIELSEN U B ET AL: "Chapter 37: Affinity maturation by chain shuffling and site directed mutagenesis", 1 January 2001, ANTIBODY ENGINEERING, SPRINGER, BERLIN, DE, PAGE(S) 515 - 539, ISBN: 978-3-540-41354-7, XP009142278
- ZHANG SHIYUN ET AL: "Predicting detection limits of enzyme-linked immunosorbent assay (ELISA) and bioanalytical techniques in general", ANALYST, vol. 139, no. 2, 1 January 2014 (2014-01-01), UK, pages 439 - 445, XP093016051, ISSN: 0003-2654, Retrieved from the Internet <URL:https://pubs.rsc.org/en/content/articlepdf/2014/an/c3an01835k> DOI: 10.1039/C3AN01835K

## Description

### Cross-Reference to Related Application

This application claims the priority of the Chinese patent application with the application No. 201811557468.5, titled "Recombinant Antibody of Anti-human N-terminal Pro-brain Natriuretic Peptide" filed to the China National Intellectual Property Administration on December 19, 2018.

### Technical Field

The present disclosure relates to the field of immune technology, in particular, to a recombinant antibody of anti-human N-terminal pro-brain natriuretic peptide.

### Background

In 1988, Japanese scholar Sudoh first isolated a polypeptide with potent diuretic, vasodilator and hypotensive effects from pig brain, and named it Brain Natriuretic Peptide (BNP). BNP is most distributed in the heart, but cardiomyocytes first synthesize proBNP containing 108 amino acids. When cardiomyocytes are stimulated, proBNP is cleaved into non-biologically active N-terminal pro-B-type natriuretic peptide (NT-proBNP) containing 76 amino acids and active B-type natriuretic peptide (BNP) containing 32 amino acids, both of which are derived from the same source and are secreted equimolarly and released into the blood circulation.

When the heart volume load increases or the cardiac function is impaired, the index concentrations of N-terminal pro-brain natriuretic peptide (NT-proBNP) and BNP will increase abnormally, wherein NT-proBNP has better biological stability, has a longer half-life ( 120 min), has relatively stable concentration, has a long effective detection time, and has about 16-20 times higher content in blood compared with BNP, therefore, it is relatively easy to detect, and the stability of its plasma samples in vitro is long (> 48 h), which is the best myocardial marker for diagnosing heart failure and evaluating cardiac function.

The content of NT-proBNP in the blood of normal people is generally less than 0.3 ng/mL. When the heart function is impaired and the myocardium expands, NT-proBNP will be rapidly synthesized and secreted in large quantities into the human blood. When finding some relevant early symptoms, accurate, sensitive, efficient and stable determination of the amount of NT-proBNP in the blood can provide a fast and accurate basis for early diagnosis for early cardiac insufficiency, heart failure, cardiogenic and non-cardiogenic heart failure with dyspnea treatment and prognosis monitoring, and acute coronary syndrome grading, etc. The current methods used to detect the content of NT-proBNP mainly include gold calibration test, fluorescence immunoassay, enzyme-linked immunosorbent assay (ELISA) and chemiluminescence microparticle immunoassays (CMIA), but these measurement methods all require a specific monoclonal antibody against NT-proBNP. However, the sensitivity and specificity of the monoclonal antibody currently used to detect NT-proBNP in China are not ideal.

The present invention is defined by the appended claims. . Relevant technologies are also known from the following documents:
WILKINS MICHAEL D. ET AL: "Quantum dot enabled lateral flow immunoassay for detection of cardiac biomarker NT-proBNP", SENSING AND BIO-SENSING RESEARCH, vol. 21, 1 November 2018 (2018-11-01), pages 46-53.
CN 106 916 225 A.
KIM HYUNG-YONG ET AL: "Affinity Maturation of Monoclonal Antibodies by Multi-Site-Directed Mutagenesis", 1 January 2014 (2014-01-01).
NIELSEN U B ET AL: "Chapter 37: Affinity maturation by chain shuffling and site directed mutagenesis", 1 January 2001 (2001-01-01).
WO 2009/066010 A1.
Zhang Shiyun ET AL: "Predicting detection limits of enzyme-linked immunosorbent assay (ELISA) and bioanalytical techniques in general", Analyst, vol. 139, no. 2, 1 January 2014 (2014-01-01), pages 439-445.

### Summary

The present disclosure relates to a novel isolated binding protein including a N-terminal pro-brain natriuretic peptide (NT-proBNP) antigen binding domain, and investigates the preparation, use and other aspects of the binding protein.

The invention is set out in the appended set of claims 1-14.

Wherein the antigen-binding domain includes six complementarity determining regions (CDR) of the following amino acid sequence;
the complementarity determining region CDR-VH1 is G-X1-S-X2-T-T-Y-Y-X3-D (SEQ ID NO:13), wherein
X1 is F, X2 is I, V or L, and X3 is I, V or L;
the complementarity determining region CDR-VH2 is M-T-K-D-X1-N-A-V-H-X2-P-T-X3-R-S (SEQ ID NO:14), wherein
X1 is G, X2 is Q or N, and X3 is I, V or L;
the complementarity determining region CDR-VH3 is V-X1-G-X2-I-D-X3-G (SEQ ID NO:15), wherein
X1 is R, X2 is I, V or L, and X3 is F or W;
the complementarity determining region CDR-VL1 is G-S-S-D-X1-V-G-X2-G-D-Y-X3-N (SEQ ID NO:16), wherein
X1 is Q or N, X2 is F or P, and X3 is I, V or L;
the complementarity determining region CDR-VL2 is I-F-X1-A-X2-S-R-X3-R-G (SEQ ID NO:17), wherein
X1 is G, X2 is T, Y or S, and X3 is I, V or L;
the complementarity determining region CDR-VL3 is G-S-X1-N-S-R-X2-Y-V-X3-G (SEQ ID NO:18), wherein
X1 is P, X2 is GG or N, and X3 is W or F;
the six complementarity determining regions have any one of the following mutation combinations:

| Site | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| Mutation combination 1 | I/I | Q/I | I/W | Q/V | T/I | P/GG |
| Mutation combination 2 | I/L | N/I | I/F | Q/L | T/L | P/N |
| Mutation combination 3 | I/V | Q/L | L/W | Q/I | T/V | A/GG |
| Mutation combination 4 | L/I | N/L | L/F | N/V | Y/I | A/N |
| Mutation combination 5 | L/L | Q/V | V/W | N/L | Y/L | G/GG |
| Mutation combination 6 | L/V | N/V | V/F | N/I | Y/V | G/N |
| Mutation combination 7 | V/I | N/V | I/W | N/I | S/I | P/N |
| Mutation combination 8 | V/L | Q/V | I/F | N/L | S/L | G/N |
| Mutation combination 9 | V/V | N/L | L/W | N/V | S/V | G/GG |
| Mutation combination 10 | I/I | Q/L | L/F | Q/I | S/I | A/GG |
| Mutation combination 11 | I/L | N/I | V/W | Q/L | S/L | A/N |
| Mutation combination 12 | I/V | Q/I | V/F | Q/V | S/V | P/GG |
| Mutation combination 13 | L/I | Q/I | I/W | Q/V | Y/I | P/GG |
| Mutation combination 14 | L/L | N/I | I/F | Q/L | Y/L | P/N |
| Mutation combination 15 | L/V | Q/L | L/W | Q/I | Y/V | A/GG |
| Mutation combination 16 | V/I | N/L | L/F | N/V | T/I | A/N |
| Mutation combination 17 | V/L | Q/V | V/W | N/L | T/L | G/GG |
| Mutation combination 18 | V/V | N/V | V/F | N/I | T/V | G/N |
| Mutation combination 19 | I/I | N/V | I/W | N/I | T/I | P/N |
| Mutation combination 20 | I/L | Q/V | I/F | N/L | T/L | G/N |
| Mutation combination 21 | I/V | N/L | L/W | N/V | T/V | G/GG |
| Mutation combination 22 | L/I | Q/L | L/F | Q/I | Y/I | A/GG |
| Mutation combination 23 | L/L | N/I | V/W | Q/L | Y/L | A/N |
| Mutation combination 24 | L/V | Q/I | V/F | Q/V | Y/V | P/GG |
| Mutation combination 25 | V/I | Q/I | I/W | Q/V | S/I | P/GG |
| Mutation combination 26 | V/L | N/I | I/F | Q/L | S/L | P/N |
| Mutation combination 27 | V/V | Q/L | L/W | Q/V | S/V | A/GG |
| Mutation combination 28 | I/I | N/L | L/F | N/V | S/I | A/N |
| Mutation combination 29 | I/L | Q/V | V/W | N/L | S/L | G/GG |
| Mutation combination 30 | I/V | N/V | V/F | N/I | S/V | G/N |
| Mutation combination 31 | L/I | N/V | I/W | N/I | T/I | P/N |
| Mutation combination 32 | L/L | Q/V | I/F | N/L | T/L | G/N |
| Mutation combination 33 | L/V | N/L | L/W | N/V | T/V | G/GG |
| Mutation combination 34 | V/I | Q/L | L/F | Q/I | Y/I | A/GG |
| Mutation combination 35 | V/L | N/I | V/W | Q/L | Y/L | A/N |
| Mutation combination 36 | V/V | Q/I | V/F | Q/V | Y/V | P/GG |
| Mutation combination 37 | I/I | Q/I | I/W | Q/V | T/I | P/GG |
| Mutation combination 38 | I/L | N/I | I/F | Q/L | T/L | A/N |
| Mutation combination 39 | I/V | Q/L | L/W | Q/I | T/V | G/GG |
| Mutation combination 40 | L/I | N/L | L/F | N/V | Y/I | G/N |
| Mutation combination 41 | L/L | Q/V | V/W | N/L | Y/L | P/N |
| Mutation combination 42 | V/I | N/V | I/W | N/I | Y/V | G/N |
| Mutation combination 43 | V/I | N/V | I/W | N/I | S/I | G/GG |
| Mutation combination 44 | V/L | Q/V | I/F | N/L | S/L | A/GG |
| Mutation combination 45 | V/V | N/L | L/W | N/V | S/V | A/N |
| Mutation combination 46 | I/I | Q/L | L/F | Q/I | S/I | P/GG |
| Mutation combination 47 | I/L | N/I | V/W | Q/L | S/L | P/GG |
| Mutation combination 48 | I/V | Q/I | V/F | Q/V | S/V | P/N |
| Mutation combination 49 | L/I | Q/I | I/W | Q/V | T/I | P/GG |
| Mutation combination 50 | L/L | N/I | I/F | Q/L | T/L | P/N |
| Mutation combination 51 | L/V | Q/L | L/W | Q/I | T/V | A/GG |
| Mutation combination 52 | V/I | N/L | L/F | N/V | Y/I | A/N |
| Mutation combination 53 | V/L | Q/V | V/W | N/L | Y/L | G/GG |
| Mutation combination 54 | V/V | N/V | V/F | N/I | Y/V | G/N |

the binding protein comprises light chain framework regions FR-L1, FR-L2, FR -L3 and FR-L4 with sequences correspondingly shown in SEQ ID NO: 1-4, and, heavy chain framework regions FR-H1, FR-H2, FR-H3 and FR-H4 with sequences correspondingly shown in SEQ ID NO: 5-8.

In one or more embodiments, the binding protein is an intact antibody including a variable region and constant region.

In one or more embodiments, the binding protein further includes an antibody constant region sequence.

In one or more embodiments, the constant region sequence is a sequence of any one constant region selected from IgG1, IgG2, IgG3, IgG4, IgA, IgM, IgE, and IgD.

In one or more embodiments, the constant region is derived from species consisted of cattle, horse, dairy cow, pig, sheep, goat, rat, mouse, dog, cat, rabbit, camel, donkey, deer, mink, chicken, duck, goose, turkey, gamecock or human.

In one or more embodiments, the constant region is derived from the sheep;
a light chain constant region sequence is shown in SEQ ID NO: 9;
a heavy chain constant region sequence is shown in SEQ ID NO: 10.

The present disclosure also relates to an isolated nucleic acid molecule, encoding the above binding protein.

The present disclosure also provides a vector, including the above nucleic acid molecule.

The present disclosure also relates to a host cell transformed with the above vector.

The present disclosure also relates to a method for producing the above binding protein, the method including the steps of:
culturing the above host cell in a culture medium and under suitable culture conditions, recovering such produced binding protein from the culture medium or from the cultured host cell.

According to an aspect of the present disclosure, the present disclosure also relates to use of the binding protein in the in vitro/ex vivo diagnosis of heart failure and evaluation of a cardiac function, and in vitro/ex vivo diagnosis of a disease related to NT-proBNP, wherein the disease related to NT-proBNP is selected from heart failure, cardiac insufficiency, cardiogenic dyspnea, pulmonary dyspnea, acute coronary syndrome, or a combination thereof; wherein, the heart failure is cardiogenic heart failure or non-cardiac heart failure.

According to an aspect of the present disclosure, the present disclosure also relates to a method for detecting NT-proBNP in a test sample, the method including:
a) contacting the NT-proBNP antigen in the test sample with the above binding protein to form an immune complex under conditions sufficient for taking an antibody/antigen binding reaction; and
b) detecting a presence of the immune complex, which indicates a presence of the NT-proBNP in the test sample.

In one or more embodiments, in step a), the immune complex further includes a second antibody that binds to the binding protein;

In one or more embodiments, in step a), the immune complex further includes a second antibody that binds to the NT-proBNP;

According to an aspect of the present disclosure, the present disclosure further relates to a kit, including the above binding protein.

The present disclosure further relates to use of the binding protein described herein in diagnosing a disease related to NT-proBNP.

The present disclosure further relates to a method for diagnosing a disease related to NT-proBNP or evaluating a cardiac function, the method including:
A) contacting a sample from a subject with the binding protein described in the present disclosure for a binding reaction under conditions sufficient for taking a binding reaction; and
B) detecting the immune complex produced by the binding reaction,
wherein the presence of the immune complex indicates the presence of a disease related to NT-proBNP or indicates the level of the cardiac function. (The subject matter does not fall within the scope of the invention as claimed.)

In one or more embodiments, the method is based on fluorescence immunoassay, chemiluminescence, colloidal gold immunoassay, radioimmunoassay, and/or enzyme-linked immunoassay.

In one or more embodiments, the sample is selected from at least one of whole blood, peripheral blood, serum, plasma or myocardial tissue.

In one or more embodiments, the subject is mammal, preferably primate, more preferably human.

In one or more embodiments, the disease related to NT-proBNP is a cardiac disease.

In one or more embodiments, the disease related to NT-proBNP is selected from heart failure, cardiac insufficiency, cardiogenic dyspnea, pulmonary dyspnea, acute coronary syndrome, or a combination thereof.

In one or more embodiments, the heart failure is cardiogenic heart failure or non-cardiac heart failure.

### Brief Description of the Drawings

In order to more clearly illustrate the specific embodiments of the present disclosure or the technical solutions in the prior art, specific embodiments or drawings that may be required in prior art descriptions are briefly described below, obviously, the drawings described below are some embodiments of the present disclosure. For those of ordinary skill in the art, other drawings can be obtained based on these drawings without creative work.

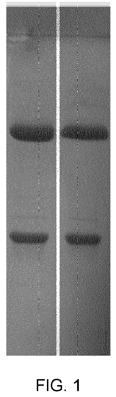

FIG. 1 is an electropherogram of a monoclonal antibody of the anti-human NT-proBNP in an embodiment of the present disclosure.

### Detailed Description of the Embodiments

The present disclosure can be more easily understood through the following description of some embodiments of the present disclosure and the detailed content of the embodiments included therein.

Before further describing the present disclosure, it should be understood that the present disclosure is not limited to the specific embodiments, because these embodiments are necessarily diverse. It should also be understood that the terms used in this specification are only to illustrate specific embodiments, rather than as limitations, because the scope of the present disclosure will only be defined in the appended claims.

### Definition

"Isolated binding protein including an antigen binding domain" broadly refers to all proteins/protein fragments including a CDR region. The term "antibody" includes a polyclonal antibody, a monoclonal antibody, and the antigen compound binding fragments of these antibodies, including Fab, F(ab')₂, Fd, Fv, scFv, a bispecific antibody and a minimum recognition unit of antibody, as well as single-chain derivatives of these antibodies and fragments. The type of antibody can select from IgG1, IgG2, IgG3, IgG4, IgA, IgM, IgE and IgD. In addition, the term "antibody" includes a naturally-occurring antibody and a non-naturally-occurring antibody, including, for example, chimeric, bifunctional, and humanized antibodies, and related synthetic isoforms. The term "antibody" can be used interchangeably with "immunoglobulin".

A "variable region" or "variable domain" of an antibody refers to an amino terminal domain of a heavy or light chain of an antibody. A variable domain of a heavy chain can be referred to as "VH". A variable domain of a light chain can be referred to as "VL". These domains are usually the most variable part of the antibody and contain the antigen binding site. A variable region of a light chain or a heavy chain (VL or VH) is consisted of three called "complementarity determining regions" or "CDRs" and the framework regions that separate the three complementarity determining regions. The scope of the framework region and CDR has been precisely defined, for example in Kabat (see "Sequences of Proteins of Immunological Interest", E. Kabat, etc., U.S. Department of Health and Human Services, (1983)) and Chothia. The framework region of the antibody plays a role in positioning and aligning the CDRs that are mainly responsible for binding to the antigen.

As used herein, "framework region", "framework regions" or "FR" means that the excluded antibody variable domains are regions other than those defined as CDRs. Each antibody variable domain framework region can be further subdivided into adjacent regions (FR1, FR2, FR3 and FR4) separated by CDRs.

Generally, the variable regions VL/VH of the heavy chain and the light chain can be obtained by arranging and linking the following numbered CDRs and FRs in the following combination: FR 1-CDR 1-FR2-CDR2-FR3-CDR3-FR4.

As used herein, the term "purified" or "isolated" associated with a polypeptide or nucleic acid means that the polypeptide or nucleic acid is not in its natural medium or in its natural form. Thus, the term "isolated" includes a polypeptide or nucleic acid taken from its original environment, for example, from the natural environment if it is naturally occurring. For example, an isolated polypeptide generally does not contain at least some proteins or other cellular components that are normally bound to it or usually mixed with it or in solution. An isolated polypeptide includes a naturally produced polypeptide contained in the cell lysate, a polypeptide in purified or partially purified form, a recombinant polypeptide, a polypeptide expressed or secreted by a cell, and a polypeptide in a heterologous host cell or the culture. Associated with nucleic acid, the term isolated or purified indicates that, for example, the nucleic acid is not in its natural genomic context (for example, in a vector, as an expression cassette, linked to a promoter, or artificially introduced into a heterologous host cell).

As used herein, the term "bispecific antibody" or "bifunctional antibody" refers to an artificial hybrid binding protein with two different pairs of heavy/light chains and two different binding sites. The bispecific binding protein can be produced by a variety of methods, including fusion of hybridomas or linking of Fab' fragments.

As used herein, the term "sequence identity" refers to the similarity between at least two different sequences. The percentage identity can be determined by standard algorithms, such as Basic Local Alignment Search Tool (BLAST); an algorithm described by Needleman et al.; or an algorithm described by Meyers et al. In one or more embodiments, a set of parameters may be Blosum 62 scoring matrix and gap penalty of 12, gap extension penalty of 4, and frameshift gap penalty of 5. In one or more embodiments, the percentage identity between two amino acid or nucleotide sequences can also be determined using the algorithm described by Meyers and Miller ((1989) CABIOS 4: 11-17), which has been incorporated to the ALIGN program (version 2.0), using the PAM120 weight residue table, gap length penalty of 12, and gap penalty of 4. Percentage identity is usually calculated by comparing sequences having similar length.

As used herein, the term "affinity" refers to the binding strength of an antigen binding domain of a binding protein or antibody to an antigen or antigen epitope. Affinity can be measured by the KD value, the smaller of which, the greater the affinity.

### Exemplary embodiments of the present disclosure

The present disclosure relates to an isolated binding protein including an N-terminal pro-brain natriuretic peptide (NT-proBNP) antigen-binding domain, wherein the antigen-binding domain includes six complementarity determining regions of the following amino acid sequences;
the complementarity determining region CDR-VH1 is G-X1-S-X2-T-T-Y-Y-X3-D, wherein X1 is **F,** X2 is I, V or L, and X3 is I, V or L;
the complementarity determining region CDR-VH2 is M-T-K-D-X1-N-A-V-H-X2-P-T-X3-R-S, wherein
X1 is G, X2 is Q or N, and X3 is I, V or L;
the complementarity determining region CDR-VH3 is V-X1-G-X2-I-D-X3-G, wherein X1 is R, X2 is I, V or L, and X3 is F or W;
the complementarity determining region CDR-VL1 is G-S-S-D-X1-V-G-X2-G-D-Y-X3-N, wherein
X1 is Q or N, X2 is F or P, and X3 is I, V or L;
the complementarity determining region CDR-VL2 is I-F-X1-A-X2-S-R-X3-R-G, wherein X1 is G, X2 is T, Y or S, and X3 is I, V or L;
the complementarity determining region CDR-VL3 is G-S-X1-N-S-R-X2-Y-V-X3-G, wherein X1 is P, X2 is GG or N, and X3 is W or F.

As used herein, the terms "N-terminal pro-brain natriuretic peptide (NT-proBNP)" and "N-terminal pro-B-type natriuretic peptide" can be used interchangeably, which refer to the non-biologically active N-terminal fragment produced by pro-brain natriuretic peptide or B-type natriuretic peptide after digestion, for example by endonuclease digestion.

the six complementarity determining regions have any one of the following mutation combinations:

| Site | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| Mutation combination 1 | I/I | Q/I | I/W | Q/V | T/I | P/GG |
| Mutation combination 2 | I/L | N/I | I/F | Q/L | T/L | P/N |
| Mutation combination 3 | I/V | Q/L | L/W | Q/I | T/V | A/GG |
| Mutation combination 4 | L/I | N/L | L/F | N/V | Y/I | A/N |
| Mutation combination 5 | L/L | Q/V | V/W | N/L | Y/L | G/GG |
| Mutation combination 6 | L/V | N/V | V/F | N/I | Y/V | G/N |
| Mutation combination 7 | V/I | N/V | I/W | N/I | S/I | P/N |
| Mutation combination 8 | V/L | Q/V | I/F | N/L | S/L | G/N |
| Mutation combination 9 | V/V | N/L | L/W | N/V | S/V | G/GG |
| Mutation combination 10 | I/I | Q/L | L/F | Q/I | S/I | A/GG |
| Mutation combination 11 | I/L | N/I | V/W | Q/L | S/L | A/N |
| Mutation combination 12 | I/V | Q/I | V/F | Q/V | S/V | P/GG |
| Mutation combination 13 | L/I | Q/I | I/W | Q/V | Y/I | P/GG |
| Mutation combination 14 | L/L | N/I | I/F | Q/L | Y/L | P/N |
| Mutation combination 15 | L/V | Q/L | L/W | Q/I | Y/V | A/GG |
| Mutation combination 16 | V/I | N/L | L/F | N/V | T/I | A/N |
| Mutation combination 17 | V/L | Q/V | V/W | N/L | T/L | G/GG |
| Mutation combination 18 | V/V | N/V | V/F | N/I | T/V | G/N |
| Mutation combination 19 | I/I | N/V | I/W | N/I | T/I | P/N |
| Mutation combination 20 | I/L | Q/V | I/F | N/L | T/L | G/N |
| Mutation combination 21 | I/V | N/L | L/W | N/V | T/V | G/GG |
| Mutation combination 22 | L/I | Q/L | L/F | Q/I | Y/I | A/GG |
| Mutation combination 23 | L/L | N/I | V/W | Q/L | Y/L | A/N |
| Mutation combination 24 | L/V | Q/I | V/F | Q/V | Y/V | P/GG |
| Mutation combination 25 | V/I | Q/I | I/W | Q/V | S/I | P/GG |
| Mutation combination 26 | V/L | N/I | I/F | Q/L | S/L | P/N |
| Mutation combination 27 | V/V | Q/L | L/W | Q/V | S/V | A/GG |
| Mutation combination 28 | I/I | N/L | L/F | N/V | S/I | A/N |
| Mutation combination 29 | I/L | Q/V | V/W | N/L | S/L | G/GG |
| Mutation combination 30 | I/V | N/V | V/F | N/I | S/V | G/N |
| Mutation combination 31 | L/I | N/V | I/W | N/I | T/I | P/N |
| Mutation combination 32 | L/L | Q/V | I/F | N/L | T/L | G/N |
| Mutation combination 33 | L/V | N/L | L/W | N/V | T/V | G/GG |
| Mutation combination 34 | V/I | Q/L | L/F | Q/I | Y/I | A/GG |
| Mutation combination 35 | V/L | N/I | V/W | Q/L | Y/L | A/N |
| Mutation combination 36 | V/V | Q/I | V/F | Q/V | Y/V | P/GG |
| Mutation combination 37 | I/I | Q/I | I/W | Q/V | T/I | P/GG |
| Mutation combination 38 | I/L | N/I | I/F | Q/L | T/L | A/N |
| Mutation combination 39 | I/V | Q/L | L/W | Q/I | T/V | G/GG |
| Mutation combination 40 | L/I | N/L | L/F | N/V | Y/I | G/N |
| Mutation combination 41 | L/L | Q/V | V/W | N/L | Y/L | P/N |
| Mutation combination 42 | V/I | N/V | I/W | N/I | Y/V | G/N |
| Mutation combination 43 | V/I | N/V | I/W | N/I | S/I | G/GG |
| Mutation combination 44 | V/L | Q/V | I/F | N/L | S/L | A/GG |
| Mutation combination 45 | V/V | N/L | L/W | N/V | S/V | A/N |
| Mutation combination 46 | I/I | Q/L | L/F | Q/I | S/I | P/GG |
| Mutation combination 47 | I/L | N/I | V/W | Q/L | S/L | P/GG |
| Mutation combination 48 | I/V | Q/I | V/F | Q/V | S/V | P/N |
| Mutation combination 49 | L/I | Q/I | I/W | Q/V | T/I | P/GG |
| Mutation combination 50 | L/L | N/I | I/F | Q/L | T/L | P/N |
| Mutation combination 51 | L/V | Q/L | L/W | Q/I | T/V | A/GG |
| Mutation combination 52 | V/I | N/L | L/F | N/V | Y/I | A/N |
| Mutation combination 53 | V/L | Q/V | V/W | N/L | Y/L | G/GG |
| Mutation combination 54 | V/V | N/V | V/F | N/I | Y/V | G/N |

the binding protein comprises light chain framework regions FR-L1, FR-L2, FR -L3 and FR-L4 with sequences correspondingly shown in SEQ ID NO: 1-4, and, heavy chain framework regions FR-H1, FR-H2, FR-H3 and FR-H4 with sequences correspondingly shown in SEQ ID NO: 5-8.

In one or more embodiments, X1s present in the six CDRs of the binding protein described in the present disclosure each independently represent the amino acid defined in the present disclosure; X2s present in the six CDRs of the binding protein described in the present disclosure each independently represent the amino acid defined in the present disclosure; X3s present in the six CDRs of the binding protein described in the present disclosure each independently represent the amino acid defined in the present disclosure.

In one or more embodiments, the binding protein is an intact antibody including a variable region and constant region.

In one or more embodiments, the binding protein is a "functional fragment" of an antibody, such as F(ab')₂, Fab', Fab, Fv, scFv, and a bispecific antibody.

scFv (sc = single chain), bispecific antibodies (diabodies).

The "functional fragment" described in the present disclosure specifically refers to an antibody fragment having identical specificity as the parent antibody for NT-proBNP. In addition to the above functional fragment, any fragments whose half-life has been increased are also included.

These functional fragments usually have identical binding specificity as the antibody from which they are derived. Those skilled in the art infer from the content recorded in the description of the present disclosure that the above functional fragment can be obtained by a method such as enzymatic digestion (including pepsin or papain) and/or a method of splitting a disulfide bond by chemical reduction from the antibody fragment of the present disclosure.

The antibody fragment can also be obtained by peptide synthesis using recombinant genetic techniques that are also known to those skilled in the art or, for example, an automatic peptide synthesizer, such as commercially available from Applied BioSystems.

It should be noted that, in addition to the amino acid sequence disclosed above in this disclosure, the species source of the framework region may be human to constitute a humanized antibody.

In one or more embodiments, the binding protein further includes an antibody constant region sequence.

In one or more embodiments, the constant region sequence is a sequence of any one constant region selected from IgG1, IgG2, IgG3, IgG4, IgA, IgM, IgE, and IgD.

In one or more embodiments, the constant region is derived from species consisting of cattle, horse, dairy cow, pig, sheep, goat, rat, mouse, dog, cat, rabbit, camel, donkey, deer, mink, chicken, duck, goose, turkey, gamecock or human.

In one or more embodiments, the constant region is derived from the sheep;
a light chain constant region sequence is shown in SEQ ID NO: 9;
a heavy chain constant region sequence is shown in SEQ ID NO: 10.

According to an aspect of the present disclosure, the present disclosure further relates to an isolated nucleic acid molecule, the nucleic acid molecule being DNA or RNA, which encodes the above binding protein.

According to an aspect of the present disclosure, the present disclosure also relates to a vector, including the above nucleic acid molecular.

The present disclosure further includes at least one nuclear construct encoding the above nucleic acid molecule, such as a plasmid, and further an expression plasmid, a construction method for which will be introduced in an embodiment of the present disclosure.

According to an aspect of the present disclosure, the present disclosure further relates to a host cell transformed with the above vector.

The host cell may be an eukaryotic cell, such as a mammalian cell.

In one or more embodiments, the host cell is a CHO cell.

According to an aspect of the present disclosure, the present disclosure further relates to a method for producing the above binding protein, the method including the steps of:
culturing the above host cell in a culture medium and under suitable culture conditions, recovering such produced binding protein from the culture medium or from the cultured host cell.

According to an aspect of the present disclosure, the present disclosure also relates to use of the binding protein in the in vitro/ex vivo diagnosis of heart failure and evaluation of a cardiac function, and in vitro/ex vivo diagnosis of a disease related to NT-proBNP, wherein the disease related to NT-proBNP is selected from heart failure, cardiac insufficiency, cardiogenic dyspnea, pulmonary dyspnea, acute coronary syndrome, or a combination thereof; wherein, the heart failure is cardiogenic heart failure or non-cardiac heart failure.

According to one aspect of the present disclosure, the present disclosure further relates to a method for detecting NT-proBNP in a test sample, the method including:
a) contacting the NT-proBNP antigen in the test sample with the above binding protein to form an immune complex under conditions sufficient for taking an antibody/antigen binding reaction; and
b) detecting a presence of the immune complex, which indicates a presence of the NT-proBNP in the test sample.

In this embodiment, the binding protein may be labeled with an indicator that shows signal intensity, so that the complex can be easily detected.

In one or more embodiments, in step a), the immune complex further includes a second antibody that binds to the binding protein;
In one or more embodiments, in step a), the immune complex further includes a second antibody that binds to the NT-proBNP;
in this embodiment, the binding protein forms a paired antibody with the second antibody in the form of a first antibody for binding to different epitopes of NT-proBNP;
the second antibody may be labeled with an indicator that shows signal intensity, so that the complex can be easily detected.

In one or more embodiments, the presence of the immune complex indicates the presence of a disease related to NT-proBNP or indicates the level of the cardiac function.

In one or more embodiments, in step a), the immune complex further includes a second antibody that binds to the NT-proBNP;

In this embodiment, the binding protein serves as an antigen of the second antibody, and the second antibody can be labeled with an indicator that shows signal intensity, so that the complex can be easily detected.

In one or more aspects the indicator for displaying signal intensity includes any one of fluorescent substance, quantum dot, digoxigenin-labeled probe, biotin, radioisotope, radiocontrast agent, paramagnetic ion fluorescent microsphere, electron dense substance, chemiluminescent marker, ultrasound contrast agent, photosensitizer, colloidal gold or enzyme.

In one or more aspects, the fluorescent substance includes any one of Alexa 350, Alexa 405, Alexa 430, Alexa 488, Alexa 555, Alexa 647, AMCA, aminoacridine, BODIPY 630/650, BODIPY 650/665, BODIPY -FL, BODIPY-R6G, BODIPY-TMR, BODIPY-TRX, 5-carboxy-4',5'-dichloro-2',7'-dimethoxyfluorescein, 5-carboxy-2',4',5',7'-tetrachlorofluorescein, 5-carboxyfluorescein, 5-carboxyrhodamine, 6-carboxyrhodamine, 6-carboxytetramethylrhodamine, Cascade Blue, Cy2, Cy3, Cy5, Cy7, 6-FAM, dansyl chloride, fluorescein, HEX, 6-JOE, NBD (7-nitrobenzo-2-oxa-1,3-diazole), Oregon Green 488, Oregon Green 500, Oregon Green514, Pacific Blue, phthalic acid, terephthalic acid, isophthalic acid, cresol solid violet, cresol blue violet, brilliant cresol blue, p-aminobenzoic acid, erythrosine, phthalocyanine, azomethine, cyanine, xanthine, succinylfluorescein, rare earth metal cryptate, tris-bispyridyldiamine europium, europium cryptate or chelate, diamine, biscyanin, La Jolla blue dye, allophycocyanin, allococyanin B, phycocyanin C, phycocyanin R, thiamine, phycoerythrin, phycoerythrin R, REG, rhodamine green, rhodamine isothiocyanate, rhodamine red, ROX, TAMRA, TET, TRIT (tetramethylrhodamine isothiol), tetramethylrhodamine and Texas Red.

In one or more aspects the radioisotope includes any one of ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁸⁶Y, ⁹⁰Y, ⁸⁹Zr, ⁹⁴mTc, ⁹⁴Tc, ⁹⁹mTc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ⁵²mMn, ⁵⁵Co, ⁷²As, ⁷⁵Br, ⁷⁶Br, ⁸²mRb and ⁸³Sr.

In one or more aspects the enzyme includes any one of horseradish peroxidase, alkaline phosphatase, and glucose oxidase.

In one or more aspects the fluorescent microsphere is a polystyrene fluorescent microsphere, and the rare earth fluorescent ion europium is wrapped inside.

According to an aspect of the present disclosure, the present disclosure further relates to a kit, including the above binding protein.

The present disclosure further relates to use of the binding protein described herein in diagnosing a disease related to NT-proBNP. (The subject matter does not fall within the scope of the invention as claimed.)

As used herein, the term "a disease related to NT-proBNP" refers to a disease in which NT-proBNP, including its protein or encoding nucleic acid, is used as a marker. In particular, in one or more embodiments of the present disclosure, a disease related to NT-proBNP may refer to a disease characterized by an increase in the level of NT-proBNP in blood, such as whole blood, plasma or serum.

The present disclosure further relates to a method for diagnosing a disease related to NT-proBNP or evaluating cardiac function, the method including:
A) contacting a sample from a subject with the binding protein described in the present disclosure for a binding reaction under conditions sufficient for taking a binding reaction; and
B) detecting the immune complex produced by the binding reaction,
wherein the presence of the immune complex indicates the presence of a disease related to NT-proBNP or indicates the level of the cardiac function. (The subject matter does not fall within the scope of the invention as claimed.)In one or more embodiments, the method is based on fluorescence immunoassay, chemiluminescence, colloidal gold immunoassay, radioimmunoassay, and/or enzyme-linked immunoassay.

In one or more embodiments, the sample is selected from at least one of whole blood, peripheral blood, serum, plasma or myocardial tissue.

In one or more embodiments, the subject is mammal, preferably primate, more preferably human.

In one or more embodiments, the disease related to NT-proBNP is a cardiac disease.

In one or more embodiments, the disease related to NT-proBNP is selected from heart failure, cardiac insufficiency, cardiogenic dyspnea, pulmonary dyspnea, acute coronary syndrome, or a combination thereof.

In one or more embodiments, the heart failure is cardiogenic heart failure or non-cardiac heart failure.

The embodiments of the present disclosure will be described in detail below together with examples, but those skilled in the art will understand that the following examples are only used to illustrate the present disclosure, and should not be regarded as limiting the scope of the present disclosure. If the specific condition is not indicated in the examples, it shall be carried out in accordance with the conventional condition or the condition recommended by the manufacturer. The reagents or instruments used without the manufacturer are all conventional products that can be purchased commercially.

### Example 1

This example provides an exemplary preparation method for a recombinant antibody of anti-human NT-proBNP.

### S1. Construction of an expression plasmid:

In this example, the restriction endonuclease and Prime Star DNA polymerase were purchased from Takara Company;
MagExtractor-RNA extraction kit was purchased from TOYOBO Company;
BD SMART^{™} RACE cDNA Amplification Kit was purchased from Takara Company;
pMD-18T vector was purchased from Takara Company;
the plasmid extraction kit was purchased from Tiangen Company;
The primer synthesis and gene sequencing were completed by Invitrogen Company;
the hybridoma cell strain that secreted the Anti-NT-proBNP 8B9 monoclonal antibody was an existing hybridoma cell strain and was resuscitated for use.

### S11, design and synthesis of primers:

Amplification of 5'RACE forward primers for heavy chains and light chains:
SMARTER II A oligonucleotide:
5'>AAGCAGTGGTATCAACGCAGAGTACXXXXX<3' (SEQ ID NO:19);
5'-RACE CDS primer (5'-CDS):5'>(T)₂₅VN<3'(N=A,C,G, or T;V=A,G,or C) (SEQ ID NO:20);
Universal Primer A mixture (UPM):
   5'>CTAATACGACTCACTATAGGGCAAGCAGTGGTATCAACGCAGAGT<3' (SEQ ID NO:21);
Nested universal primer A (NUP):
   5'>AAGCAGTGGTATCAACGCAGAGT<3' (SEQ ID NO:22);
mkR: 5'>CCCGAATTCCTAAGAACACTCTGAGGGCTTCACTG<3' (SEQ ID NO:23);
mHR: 5'>CCCGAATTCTCATTTACCCGGAGGCTTAGAGAT<3' (SEQ ID NO:24).

### S12, Gene cloning and sequencing for antibody variable region:

The RNA was extracted from the hybridoma cell strain secreting Anti-NT-proBNP 8B9 monoclonal antibody, and the first chain cDNA was synthesized using the SMARTERTM RACE cDNA Amplification Kit and the SMARTER II A oligonucleotide and 5'-CDS primers in the kit. The obtained first chain cDNA product was used as a template for PCR amplification. Light chain genes were amplified with universal primer A mix (UPM), nested universal primer A (NUP) and mkR, and heavy chain genes were amplified with universal primer A mix (UPM), nested universal primer A (NUP) and mHR. Wherein, the primer pair for the light chain amplified the target band of about 0.7 KB, and the primer pair for the heavy chain amplified the target band of about 1.4 KB. The product purified and recovered by agarose gel electrophoresis was subjected to A-addition reaction with rTaq DNA polymerase, followed by inserting into pMD-18T vector and transforming into DH5α competent cells. After the growth of the colony, 4 clones of the heavy chain and light chain gene clones were picked and sent to Invitrogen Company for sequencing.

### S13, Sequence analysis of variable region genes of anti-NT-proBNP 8B9 antibody:

The gene sequence obtained by the above sequencing was analyzed in the IMGT antibody database, and the amplified genes were analyzed to confirm that the heavy chain and light chain primers were correct, and the light chain amplified genes by using VNTI11.5 software. Among the gene fragments amplified by the light chain, the VL gene sequence was 420 bp, belonging to the Vkll gene family, with a 60 bp leader peptide sequence in front of it; among the gene fragments amplified by the heavy chain primer pair, the VH gene sequence was 402 bp, belonging to the VH1 gene family, with a 57 bp leader peptide sequence in front of it.

### S14, Construction of recombinant antibody expression plasmid:

pcDNA^{™} 3.4 TOPO^{®} vector was a constructed recombinant antibody eukaryotic expression vector. The expression vector had been introduced with Hindlll, BamHI, EcoRI and other polyclonal restriction sites, named as pcDNA3.4A expression vector, and then referred to as 3.4A expression vector; according to the above sequencing results of antibody variable region genes in pMD-18T, the VL and VH gene-specific primers of Anti-NT-proBNP 8B9 antibody were designed, with Hindlll and EcoRI restriction sites and protective bases at both terminals, with the primers as follows:
NT-proBNP 8B9-HF:
5'>CCCAAGCTTGCCACCATGAACCCACTGTGGACCCTCCTCTTT<3' (SEQ ID NO:25);
NT-proBNP 8B9-HR:
   5'>CCCGAATTCTCATTTACCCGGAGGCTTAGAGAT<3' (SEQ ID NO:26);
NT-proBNP 8B9-LF:
   5'>CCCAAGCTTGCCACCATGGCCTGGTCCCCTCTGCTCCTCAC<3' (SEQ ID NO:27);
NT-proBNP 8B9-LR:
   5'>CCCGAATTCCTAAGAACACTCTGAGGGCTTCACTG<3' (SEQ ID NO:28);

The 0.7 KB light chain gene fragment and 1.4 kb heavy chain gene fragment were amplified by PCR amplification method. The heavy chain and light chain gene fragments were digested with Hindlll/EcoRI, respectively, and the 3.4A vector was digested with Hindlll/EcoRl. After the fragment and vector being purified and recovered, the heavy chain gene and light chain gene were linked to the 3.4A expression vector, respectively, to obtain recombinant expression plasmids of heavy chain and light chain.

### S2. Screening of stable cell strains

### S21 Recombinant antibody expression plasmid was transiently transfected into CHO cells to confirm the activity

Plasmids were diluted with ultrapure water to 400 ng/ml, and the CHO cells were adjusted to 1.43×107cells /ml in a centrifuge tube. 100 µl of plasmids were mixed with 700 µl of cells, which were transferred to the electrotransfection cuvette for electrotransfection. The samples were taken and counted on Day 3, 5 and 7, and were collected on Day 7 for testing.

Recombinant NT antigen (self-produced, 161213) was diluted with coating solution to the specified concentration at 100 µL per well overnight at 4°C; which was washed twice with washing solution and patted dry on the next day; with addition of blocking solution (20%BSA+80%PBS) at 120 µL per well at 37°C for 1 h and patted dry; added the diluted cell supernatant, 100 µL/well at 37°C for 30 min (partial supernatant for 1 h); washed 5 times with washing solution and patted dry; added rabbit anti-goat IgG-HRP 100 µL per well at 37°C for 30 min; washed 5 times with washing solution and patted dry; added color developing solution A (50 µL/well), added color developing solution B (50 µL/well) for 10 min; added stop solution, 50 µL/well; followed by reading the OD value at 450nm (reference 630 nm) on the microplate reader. The results showed that the OD of the reaction was still greater than 1.0 after the cell supernatant being diluted 1000 times, and the reaction OD of the wells without the cell supernatant was less than 0.1, indicating that the antibody produced after transient transfection with the plasmid was active against recombinant NT protein.

### S22 Linearization of recombinant antibody expression plasmid

Preparation of the following reagents: Buffer 50 µl, DNA 100 µg/tube, Puv I enzyme 10 µl, supplemented with sterile water to 500 µl, for digestion overnight at 37°C in the water bath; which was extracted with an equal volume of phenol/chloroform/isoamyl alcohol (lower layer) 25:24 :1 and then chloroform (aqueous phase) in sequence; precipitated on ice with 0.1 times volume (aqueous phase) 3M sodium acetate and 2 times volume of ethanol, and the obtained precipitate was rinsed with 70% ethanol to remove the organic solvent, followed by thawing with an appropriate amount of sterilized water until the ethanol evaporated completely to be determined the concentration finally.

### S23 Stable transfection of recombinant antibody expression plasmid and selection of stable cell strains under pressure

Plasmids were diluted with ultrapure water to 400 ng/ml, and the CHO cells were adjusted to 1.43×10⁷cells/ml in a centrifuge tube. 100 µl of plasmids were mixed with 700 µl of cells, which were transferred to the electrotransfection cuvette for electrotransfection and counted on the next day, followed by culturing at 25 µmol/L MSX 96 wells under pressure for about 25 days.

The clonal wells with labeled cells were observed under a microscope, and recorded the confluence; from which the culture supernatant were taken and sent for testing; and cell strains with high antibody concentration and relative concentration were transferred to 24 wells, and then transferred to 6 wells in about 3 days; followed by preservation and batch culture with adjustment the cell density to 0.5×10⁶cells/ml, from which 2.2 ml for batch culture at the cell density of 0.3×10⁶cells/ml, and 2 ml for preservation; and the supernatant from 6 wells batch culture for 7 days was sent for testing, from which the cell strain with smaller antibody concentration and cell diameter was selected for TPP preservation and passage.

### S3. Production of recombinant antibody

### S31 Cell expansion culture

After resuscitation, the cells were cultured in a 125 ml shake flask with a 30 ml inoculation volume and 100% Dynamis medium, placed in a shaker with a rotation speed of 120 r/min at 37°C, and a carbon dioxide of 8%. Cells were cultured for 72 h and inoculated at an inoculation density of 500,000 cells/ml for expansion culture, in which the expansion volume was calculated according to production requirements, using 100% Dynamis medium. Then the culture was expanded every 72 h. When the cell mass met the production requirements, the inoculation density was strictly controlled to about 500,000 cells/ml for production.

### S32 Production in the shake flask and purification

Shake flask parameters: rotation at a speed of 120 r/min, at a temperature of 37°C, and with carbon dioxide of 8%. Feeding: feeding every day was started at after it is cultured for 72 h in the shake flask. HyCloneTM Cell BoostTM Feed 7a was fed 3% of the initial culture volume every day, and Feed 7b with fed every thousandth of the initial culture volume, until to Day 12 (feeding on Day 12). Glucose was supplemented at 3g/L on Day 6. Samples were collected on Day 13. Then the affinity purification was carried out by a proteinA affinity chromatography column. 4 µg of purified antibodies was taken for reducibility SDS-PAGE, and 4 µg of foreign control antibodies was used as a control. The electrophoretogram was shown in FIG. 1. After reducibility SDS-PAGE, two bands were displayed, one Mr was 50 KD (heavy chain), and the other Mr was 28 KD (light chain).

### Example 2

### Antibody affinity analysis and activity identification

The antibody obtained in Example 1 was analyzed to have a light chain as shown in SEQ ID NO: 11 and a heavy chain as shown in SEQ ID NO: 12.

After analysis, the complementarity determining region (WT) of the heavy chain:
CDR-VH1 was G-P(X1)-S-I(X2)-T-T-Y-Y-I(X3)-D;
CDR-VH2 was M-T-K-D-A(X1)-N-A-V-H-Q(X2)-P-T-I(X3)-R-S;
CDR-VH3 was V-K(X1)-G-I(X2)-I-D-W(X3)-G;
the complementarity determining region of the light chain:
CDR-VL1 was G-S-S-D-Q(X1)-V-G-P(X2)-G-D-Y-V(X3)-N;
CDR-VL2 was I-F-A(X1)-A-T(X2)-S-R-I(X3)-R-G;
CDR-VL3 was G-S-P(X1)-N-S-R-GG(X2)-Y-V-W(X3)-G;
wherein, X1, X2, and X3 were all mutation sites. Table 1 Mutation sites related to antibody activity

| Site | CDR-VH1 X1 | CDR-VH2 X1 | CDR-VH3 X1 | CDR-VL1 X2 | CDR-VL2 X1 | CDR-VL3 X3 |
|---|---|---|---|---|---|---|
| WT | P | A | K | P | A | w |
| Mutation 1 | F | G | R | F | G | F |
| Mutation 2 | P | G | R | P | G | F |
| Mutation 3 | F | A | R | F | A | F |
| Mutation 4 | P | G | K | F | G | w |

The CDR sites in WT was mutated by the inventor as described above to obtain antibodies with better activity.

Recombinant NT antigen was diluted with coating solution to 1 µg/ml in a microplate overnight at 4°C; which was washed twice with washing solution and patted dry on the next day; with addition of blocking solution (20%BSA+80%PBS) at 120 µL per well at 37°C for 1 h and patted dry; added the diluted NT monoclonal antibody, 100 µL/well at 37°C for 30 min (partial supernatant for 1 h); washed 5 times with washing solution and patted dry; added rabbit anti-goat IgG-HRP 100 µL per well at 37°C for 30 min; washed 5 times with washing solution and patted dry; added color developing solution A (50 µL/well), added color developing solution B (50 µL/well) for 10 min; added stop solution, 50 µL/well; followed by reading the OD value at 450 nm (reference 630 nm) on the microplate reader.

**Table 2 Antibody activity analysis data**

| Sample concentration ng/ml | WT | Mutation 1 | Mutation 2 | Mutation 3 | Mutation 4 |
|---|---|---|---|---|---|
| 111.11 | 1.528 | 1.827 | 1.772 | 1.764 | 1.724 |
| 37.04 | 1.236 | 1.568 | 1.526 | 1.533 | 1.501 |
| 12.35 | 0.732 | 1.110 | 1.031 | 1.022 | 0.959 |
| 4.12 | 0.312 | 0.564 | 0.523 | 0.520 | 0.489 |
| 1.37 | 0.218 | 0.355 | 0.353 | 0.347 | 0.300 |
| 0 | 0.067 | 0.121 | 0.089 | 0.076 | 0.115 |

From above tables, mutation 1 was used as the framework sequence to screen for mutation sites with better potency due to having best activity (ensure that the antibody activity obtained by screening was similar to that of mutation 1, having the antibody activity of ±10 %), some of the results were as follows.

**Table 3 Mutation sites related to antibody affinity**

| Site | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| Mutation 1 | I/I | Q/I | I/W | Q/V | T/I | P/GG |
| Mutation 1-1 | I/L | N/I | I/F | Q/L | T/L | P/N |
| Mutation 1-2 | I/V | Q/L | L/W | Q/I | T/V | A/GG |
| Mutation 1-3 | L/I | N/L | L/F | N/V | Y/I | A/N |
| Mutation 1-4 | L/L | Q/V | V/W | N/L | Y/L | G/GG |
| Mutation 1-5 | L/V | N/V | V/F | N/I | Y/V | G/N |
| Mutation 1-6 | V/I | N/V | I/W | N/I | S/I | P/N |
| Mutation 1-7 | V/L | Q/V | I/F | N/L | S/L | G/N |
| Mutation 1-8 | V/V | N/L | L/W | N/V | S/V | G/GG |
| Mutation 1-9 | I/I | Q/L | L/F | Q/I | S/I | A/GG |
| Mutation 1-10 | I/L | N/I | V/W | Q/L | S/L | A/N |
| Mutation 1-11 | I/V | Q/I | V/F | Q/V | S/V | P/GG |
| Mutation 1-12 | L/I | Q/I | I/W | Q/V | Y/I | P/GG |
| Mutation 1-13 | L/L | N/I | I/F | Q/L | Y/L | P/N |
| Mutation 1-14 | L/V | Q/L | L/W | Q/I | Y/V | A/GG |
| Mutation 1-15 | V/I | N/L | L/F | N/V | T/I | A/N |
| Mutation 1-16 | V/L | Q/V | V/W | N/L | T/L | G/GG |
| Mutation 1-17 | V/V | N/V | V/F | N/I | T/V | G/N |
| Mutation 1-18 | I/I | N/V | I/W | N/I | T/I | P/N |
| Mutation 1-19 | I/L | Q/V | I/F | N/L | T/L | G/N |
| Mutation 1-20 | I/V | N/L | L/W | N/V | T/V | G/GG |
| Mutation 1-21 | L/I | Q/L | L/F | Q/I | Y/I | A/GG |
| Mutation 1-22 | L/L | N/I | V/W | Q/L | Y/L | A/N |
| Mutation 1-23 | L/V | Q/I | V/F | Q/V | Y/V | P/GG |
| Mutation 1-24 | V/I | Q/I | I/W | Q/V | S/I | P/GG |
| Mutation 1-25 | V/L | N/I | I/F | Q/L | S/L | P/N |
| Mutation 1-26 | V/V | Q/L | L/W | Q/V | S/V | A/GG |
| Mutation 1-27 | I/I | N/L | L/F | N/V | S/I | A/N |
| Mutation 1-28 | I/L | Q/V | V/W | N/L | S/L | G/GG |
| Mutation 1-29 | I/V | N/V | V/F | N/I | S/V | G/N |
| Mutation 1-30 | L/I | N/V | I/W | N/I | T/I | P/N |
| Mutation 1-31 | L/L | Q/V | I/F | N/L | T/L | G/N |
| Mutation 1-32 | L/V | N/L | L/W | N/V | T/V | G/GG |
| Mutation 1-33 | V/I | Q/L | L/F | Q/I | Y/I | A/GG |
| Mutation 1-34 | V/L | N/I | V/W | Q/L | Y/L | A/N |
| Mutation 1-35 | V/V | Q/I | V/F | Q/V | Y/V | P/GG |
| Mutation 1-36 | I/I | Q/I | I/W | Q/V | T/I | P/GG |
| Mutation 1-37 | I/L | N/I | I/F | Q/L | T/L | A/N |
| Mutation 1-38 | I/V | Q/L | L/W | Q/I | T/V | G/GG |
| Mutation 1-39 | L/I | N/L | L/F | N/V | Y/I | G/N |
| Mutation 1-40 | L/L | Q/V | V/W | N/L | Y/L | P/N |
| Mutation 1-41 | V/I | N/V | I/W | N/I | Y/V | G/N |
| Mutation 1-42 | V/I | N/V | I/W | N/I | S/I | G/GG |
| Mutation 1-43 | V/L | Q/V | I/F | N/L | S/L | A/GG |
| Mutation 1-44 | V/V | N/L | L/W | N/V | S/V | A/N |
| Mutation 1-45 | I/I | Q/L | L/F | Q/I | S/I | P/GG |
| Mutation 1-46 | I/L | N/I | V/W | Q/L | S/L | P/GG |
| Mutation 1-47 | I/V | Q/I | V/F | Q/V | S/V | P/N |
| Mutation 1-48 | L/I | Q/I | I/W | Q/V | T/I | P/GG |
| Mutation 1-49 | L/L | N/I | I/F | Q/L | T/L | P/N |
| Mutation 1-50 | L/V | Q/L | L/W | Q/I | T/V | A/GG |
| Mutation 1-51 | V/I | N/L | L/F | N/V | Y/I | A/N |
| Mutation 1-52 | V/L | Q/V | V/W | N/L | Y/L | G/GG |
| Mutation 1-53 | V/V | N/V | V/F | N/I | Y/V | G/N |

### Affinity analysis

Data from the enzyme immunoassay indirect method were analyzed in the same way as the activity identification, with four gradients of 3 µg/ml, 1.5 µg/ml, 0.75 µg/ml and 0.375 µg/ml for coating; the antibody was diluted from 1000 ng/ml by 2 times to 0.977 ng/ml for loading sample. OD values corresponding to different antibody concentrations at different coating concentrations were obtained. Under the same coating concentration, data were plotted logarithmically with the antibody concentration as the abscissa and the OD value as the ordinate. According to the fitting equation, the antibody concentration at 50% of the maximum OD value was calculated, which was used into the formula: K=(n-1)/(2×(n×Ab'-Ab)) to calculate the reciprocal of the affinity constant, wherein Ab and Ab' represented the antibody concentration at 50% of the maximum OD value at the corresponding coating concentration (Ag, Ag'), n=Ag/Ag'; every two coating concentrations could be combined to calculate a K value, and finally six K values could be obtained, which were averaged followed by being calculated their reciprocals to obtain the affinity constant KD.

**Table 4 Affinity analysis data**

| No. | K_{D} (M) | No. | K_{D} (M) |
|---|---|---|---|
| Mutation 1 | 1.73E-09 | Mutation 1-28 | 2.25E-09 |
| Mutation 1-1 | 2.51E-09 | Mutation 1-29 | 1.49E-09 |
| Mutation 1-2 | 2.61E-09 | Mutation 1-30 | 2.66E-09 |
| Mutation 1-3 | 1.52E-09 | Mutation 1-31 | 1.84E-09 |
| Mutation 1-4 | 2.07E-09 | Mutation 1-32 | 1.52E-09 |
| Mutation 1-5 | 6.10E-09 | Mutation 1-33 | 2.60E-09 |
| Mutation 1-6 | 1.17E-09 | Mutation 1-34 | 3.12E-09 |
| Mutation 1-7 | 1.53E-09 | Mutation 1-35 | 1.51E-09 |
| Mutation 1-8 | 2.22E-09 | Mutation 1-36 | 5.64E-09 |
| Mutation 1-9 | 1.56E-09 | Mutation 1-37 | 1.57E-09 |
| Mutation 1-10 | 3.20E-09 | Mutation 1-38 | 1.82E-09 |
| Mutation 1-11 | 1.52E-09 | Mutation 1-39 | 2.45E-09 |
| Mutation 1-12 | 2.75E-09 | Mutation 1-40 | 3.47E-09 |
| Mutation 1-13 | 2.09E-09 | Mutation 1-41 | 2.82E-09 |
| Mutation 1-14 | 8.75E-10 | Mutation 1-42 | 2.22E-09 |
| Mutation 1-15 | 1.76E-09 | Mutation 1-43 | 2.93E-09 |
| Mutation 1-16 | 3.27E-09 | Mutation 1-44 | 1.26E-09 |
| Mutation 1-17 | 3.39E-09 | Mutation 1-45 | 1.53E-09 |
| Mutation 1-18 | 1.73E-09 | Mutation 1-46 | 3.49E-09 |
| Mutation 1-19 | 1.51E-09 | Mutation 1-47 | 2.79E-09 |
| Mutation 1-20 | 2.61E-09 | Mutation 1-48 | 2.02E-09 |
| Mutation 1-21 | 2.53E-09 | Mutation 1-49 | 6.83E-09 |
| Mutation 1-22 | 2.07E-09 | Mutation 1-50 | 2.16E-09 |
| Mutation 1-23 | 8.10E-10 | Mutation 1-51 | 9.44E-10 |
| Mutation 1-24 | 1.17E-09 | Mutation 1-52 | 2.71E-09 |
| Mutation 1-25 | 2.16E-09 | Mutation 1-53 | 2.06E-09 |
| Mutation 1-26 | 1.51E-09 | | |
| Mutation 1-27 | 8.55E-10 | | |

From Table 4, the mutation sites listed in Table 3 had little effect on the affinity of the antibody.

In order to verify the above results, the above experiment was repeated with WT as the framework sequence to verify the affinity of the mutation site, with some of the results as follows.

**Table 5 Mutations with WT as the framework**

| Site | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| WT | I/I | Q/I | I/F | Q/V | T/I | P/GG |
| WT 1-1 | V/I | N/I | V/W | N/I | Y/I | A/N |
| WT 1-2 | L/I | Q/L | V/W | N/L | S/I | P/N |
| WT 1-3 | I/L | Q/I | L/F | N/V | T/I | A/N |
| WT 1-4 | V/V | Q/L | V/F | Q/V | T/I | G/GG |
| WT 1-5 | L/L | N/V | L/F | Q/L | S/I | A/N |
| WT 1-6 | I/V | Q/I | L/W | Q/L | S/L | A/GG |
| WT 1-7 | V/L | Q/V | V/W | N/I | Y/L | G/GG |
| WT 1-8 | L/V | Q/V | I/W | N/I | Y/V | P/N |
| WT 1-9 | V/I | Q/L | L/W | N/V | Y/L | G/GG |

**Table 6 Affinity analysis data**

| No. | KD (M) | No. | KD (M) |
|---|---|---|---|
| WT | 3.02E-09 | WT 1-5 | 2.26E-08 |
| WT 1-1 | 1.78E-08 | WT 1-6 | 6.14E-09 |
| WT 1-2 | 3.48E-09 | WT 1-7 | 5.05E-09 |
| WT 1-3 | 2.53E-09 | WT 1-8 | 4.78E-09 |
| WT 1-4 | 3.02E-09 | WT 1-9 | 2.47E-09 |

According to the analysis from Table 5 and Table 6, the above mutation site had little correlation with other sites, provided that the activity of the antibody was ensured.

### Example 3 Stability analysis

The antibody was placed at 4°C (fridge), -80°C (fridge), 37°C (incubator) for 21 days, with taking samples at Day 7, Day 14 and Day 21 for state observation, and the activities of samples at Day 7 were detected. The results showed that under the three assessment conditions, there was no significant protein status change after the antibody was placed for 21 days, and the activity did not decrease with the increasement of the assessment temperature, indicating that the self-produced antibody was stable. The following table showed the OD results of enzyme immunoassay test for 21 days for mutation 1.

**Table 7 Stability analysis data**

| Sample concentration (ng/ml) | 111.11 | 12.35 | 0 |
|---|---|---|---|
| 4°C , sample on Day 21 | 1.726 | 0.864 | 0.047 |
| -80°C , sample on Day 21 | 1.845 | 0.976 | 0.036 |
| 37°C , sample on Day 21 | 1.813 | 0.843 | 0.033 |

Furthermore, 8 randomly selected mutant antibodies were tested for stability; the above antibodies were stored at 37°C for 72 hours, and after removal, they were tested with the same negative and positive quality control samples under the same test conditions with the same batch of antibodies stored at 4°C for 72 hours. The test method was the same as the antibody activity analysis method used in the above examples. The linearity of each group of antibodies could reach more than 99.50%, and the CV value was less than 10%, which showed that the above antibodies had excellent stability.

### Example 4 Evaluation for pairing performance

The above antibodies in Table 4 with another internal antibody (antibody paired with the original WT sequence antibody) were verified using a paired antibody experiment by the applicant. Their specificities maintained at the original high level verified by the double antibody sandwich method paired experiment, but due to the increased activity and affinity of the mutant antibody, it exhibited higher sensitivity.

The mutant 1 antibody and WT antibody were used as coating antibodies, accompanied with another strain as a labeled NT-oroBNP antibody. The performance difference was compared on the chemiluminescence evaluation platform, with the specific performance shown in the following table:

**Table 8 Evaluation table for pairing performance**

| Performance index | Decection range | Sensitivity | Linear | Detection rate |
|---|---|---|---|---|
| Mutation 1 | 0-50000pg/ml | 5.8pg/ml | 0.97689 | 100% |
| WT | 0-50000pg/ml | 6pg/ml | 0.95638 | 99% |

Finally, it should be noted that the above embodiments are only used to illustrate the technical solutions of the present disclosure, not to limit them; although the present disclosure has been described in detail with reference to the foregoing embodiments, but those of ordinary skill in the art should understand that: the technical solutions recorded in the foregoing embodiments can still be modified, or some or all of the technical features can be equivalently replaced; and these modifications or replacements do not cause the essence of the corresponding technical solutions to deviate from the scope of the technical solutions of the embodiments in the present disclosure.

### Industrial Applicability

The binding protein described in the present disclosure has strong activity and high affinity to human NT-proBNP. The binding protein described in the present disclosure can be used to diagnose a disease related to NT-proBNP, such as heart failure, and can be used to evaluate cardiac function with high sensitivity and specificity.

## Claims

1. An isolated binding protein comprising an N-terminal pro-brain natriuretic peptide antigen-binding domain, wherein the antigen-binding domain comprises six complementarity determining regions (CDR) of the following amino acid sequences;
the complementarity determining region CDR-VH1 is G-X1-S-X2-T-T-Y-Y-X3-D, wherein
X1 is **F,** X2 is I, V or L, and X3 is I, V or L;
the complementarity determining region CDR-VH2 is M-T-K-D-X1-N-A-V-H-X2-P-T-X3-R-S, wherein
X1 is G, X2 is Q or N, and X3 is I, V or L;
the complementarity determining region CDR-VH3 is V-X1-G-X2-I-D-X3-G, wherein
X1 is R, X2 is I, V or L, and X3 is F or W;
the complementarity determining region CDR-VL1 is G-S-S-D-X1-V-G-X2-G-D-Y-X3-N, wherein
X1 is Q or N, **X2 is F,** and X3 is I, V or L;
the complementarity determining region CDR-VL2 is I-F-X1-A-X2-S-R-X3-R-G, wherein
X1 is G, X2 is T, Y or S, and X3 is I, V or L;
the complementarity determining region CDR-VL3 is G-S-X1-N-S-R-X2-Y-V-X3-G, wherein
X1 is P, A or G, X2 is GG or N, and X3 is F; the six complementarity determining regions have any one of the following mutation combinations:
| Site | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| Mutation combination 1 | I/I | Q/I | I/W | Q/V | T/I | P/GG |
| Mutation combination 2 | I/L | N/I | I/F | Q/L | T/L | P/N |
| Mutation combination 3 | I/V | Q/L | L/W | Q/I | T/V | A/GG |
| Mutation combination 4 | L/I | N/L | L/F | N/V | Y/I | A/N |
| Mutation combination 5 | L/L | Q/V | V/W | N/L | Y/L | G/GG |
| Mutation combination 6 | L/V | N/V | V/F | N/I | Y/V | G/N |
| Mutation combination 7 | V/I | N/V | I/W | N/I | S/I | P/N |
| Mutation combination 8 | V/L | Q/V | I/F | N/L | S/L | G/N |
| Mutation combination 9 | V/V | N/L | L/W | N/V | S/V | G/GG |
| Mutation combination 10 | I/I | Q/L | L/F | Q/I | S/I | A/GG |
| Mutation combination 11 | I/L | N/I | V/W | Q/L | S/L | A/N |
| Mutation combination 12 | I/V | Q/I | V/F | Q/V | S/V | P/GG |
| Mutation combination 13 | L/I | Q/I | I/W | Q/V | Y/I | P/GG |
| Mutation combination 14 | L/L | N/I | I/F | Q/L | Y/L | P/N |
| Mutation combination 15 | L/V | Q/L | L/W | Q/I | Y/V | A/GG |
| Mutation combination 16 | V/I | N/L | L/F | N/V | T/I | A/N |
| Mutation combination 17 | V/L | Q/V | V/W | N/L | T/L | G/GG |
| Mutation combination 18 | V/V | N/V | V/F | N/I | T/V | G/N |
| Mutation combination 19 | I/I | N/V | I/W | N/I | T/I | P/N |
| Mutation combination 20 | I/L | Q/V | I/F | N/L | T/L | G/N |
| Mutation combination 21 | I/V | N/L | L/W | N/V | T/V | G/GG |
| Mutation combination 22 | L/I | Q/L | L/F | Q/I | Y/I | A/GG |
| Mutation combination 23 | L/L | N/I | V/W | Q/L | Y/L | A/N |
| Mutation combination 24 | L/V | Q/I | V/F | Q/V | Y/V | P/GG |
| Mutation combination 25 | V/I | Q/I | I/W | Q/V | S/I | P/GG |
| Mutation combination 26 | V/L | N/I | I/F | Q/L | S/L | P/N |
| Mutation combination 27 | V/V | Q/L | L/W | Q/V | S/V | A/GG |
| Mutation combination 28 | I/I | N/L | L/F | N/V | S/I | A/N |
| Mutation combination 29 | I/L | Q/V | V/W | N/L | S/L | G/GG |
| Mutation combination 30 | I/V | N/V | V/F | N/I | S/V | G/N |
| Mutation combination 31 | L/I | N/V | I/W | N/I | T/I | P/N |
| Mutation combination 32 | L/L | Q/V | I/F | N/L | T/L | G/N |
| Mutation combination 33 | L/V | N/L | L/W | N/V | T/V | G/GG |
| Mutation combination 34 | V/I | Q/L | L/F | Q/I | Y/I | A/GG |
| Mutation combination 35 | V/L | N/I | V/W | Q/L | Y/L | A/N |
| Mutation combination 36 | V/V | Q/I | V/F | Q/V | Y/V | P/GG |
| Mutation combination 37 | I/I | Q/I | I/W | Q/V | T/I | P/GG |
| Mutation combination 38 | I/L | N/I | I/F | Q/L | T/L | A/N |
| Mutation combination 39 | I/V | Q/L | L/W | Q/I | T/V | G/GG |
| Mutation combination 40 | L/I | N/L | L/F | N/V | Y/I | G/N |
| Mutation combination 41 | L/L | Q/V | V/W | N/L | Y/L | P/N |
| Mutation combination 42 | V/I | N/V | I/W | N/I | Y/V | G/N |
| Mutation combination 43 | V/I | N/V | I/W | N/I | S/I | G/GG |
| Mutation combination 44 | V/L | Q/V | I/F | N/L | S/L | A/GG |
| Mutation combination 45 | V/V | N/L | L/W | N/V | S/V | A/N |
| Mutation combination 46 | I/I | Q/L | L/F | Q/I | S/I | P/GG |
| Mutation combination 47 | I/L | N/I | V/W | Q/L | S/L | P/GG |
| Mutation combination 48 | I/V | Q/I | V/F | Q/V | S/V | P/N |
| Mutation combination 49 | L/I | Q/I | I/W | Q/V | T/I | P/GG |
| Mutation combination 50 | L/L | N/I | I/F | Q/L | T/L | P/N |
| Mutation combination 51 | L/V | Q/L | L/W | Q/I | T/V | A/GG |
| Mutation combination 52 | V/I | N/L | L/F | N/V | Y/I | A/N |
| Mutation combination 53 | V/L | Q/V | V/W | N/L | Y/L | G/GG |
| Mutation combination 54 | V/V | N/V | V/F | N/I | Y/V | G/N |
the binding protein comprises light chain framework regions FR-L1, FR-L2, FR -L3 and FR-L4 with sequences correspondingly shown in SEQ ID NO: 1-4, and, heavy chain framework regions FR-H1, FR-H2, FR-H3 and FR-H4 with sequences correspondingly shown in SEQ ID NO: 5-8.

2. The isolated binding protein comprising an N-terminal pro-brain natriuretic peptide antigen-binding domain according to claim 1, wherein the binding protein is one of F(ab')₂, Fab', Fab, Fv, scFv, and a bispecific antibody .

3. The isolated binding protein comprising an N-terminal pro-brain natriuretic peptide antigen-binding domain according to any one of claims 1 to 2, wherein the binding protein further comprises a constant region sequence of antibody;
preferably, the constant region sequence is a sequence of any one constant region selected from IgG1, IgG2, IgG3, IgG4, IgA, IgM, IgE, and IgD;
preferably, the constant region is derived from species consisting of cattle, horse, pig, sheep, goat, rat, mouse, dog, cat, rabbit, camel, donkey, deer, mink, chicken, duck, goose or human;
preferably, the constant region is derived from the sheep;
a light chain constant region sequence is shown in SEQ ID NO: 9;
a heavy chain constant region sequence is shown in SEQ ID NO: 10;
preferably the binding protein is labeled by an indicator which shows signal intensity.

4. An isolated nucleic acid molecule, the nucleic acid molecule being DNA or RNA, which encodes the binding protein according to any one of claims 1-3.

5. A vector, comprising the nucleic acid according to claim 4.

6. A host cell transformed with the vector according to claim 5.

7. A method for producing the binding protein according to any one of claims 1-3, the method comprising the steps of:
culturing the host cell according to claim 6 in a culture medium and under suitable culture conditions, recovering such produced binding protein from the culture medium or from the cultured host cell.

8. Use of the binding protein according to any one of claims 1-3 in the in vitro/ex vivo diagnosis of heart failure and evaluation of a cardiac function, and in vitro/ex vivo diagnosis of a disease related to NT-proBNP, wherein the disease related to NT-proBNP is selected from heart failure, cardiac insufficiency, cardiogenic dyspnea, pulmonary dyspnea, acute coronary syndrome, or a combination thereof;
wherein, the heart failure is cardiogenic heart failure or non-cardiac heart failure.

9. A method for detecting NT-proBNP in a test sample, the method comprising:
a) contacting the NT-proBNP antigen in the test sample with the binding protein according to any one of claims 1-3 to form an immune complex under conditions sufficient for taking an antibody/antigen binding reaction; and
b) detecting a presence of the immune complex, which indicates a presence of the NT-proBNP in the test sample;
preferably, in step a), the immune complex further comprises a second antibody that binds to the binding protein;
preferably, in step a), the immune complex further comprises a second antibody that binds to the NT-proBNP;
preferably, the presence of the immune complex indicates the presence of a disease related to NT-proBNP or indicates the level of the cardiac function.

10. The method according to claim 9, wherein the method is based on fluorescence immunoassay, chemiluminescence, colloidal gold immunoassay, radioimmunoassay, and/or enzyme-linked immunoassay.

11. The method according to claim 9 or 10, wherein the test sample is selected from at least one of whole blood, peripheral blood, serum, plasma or myocardial tissue.

12. The method according to any one of claims 9-11, wherein a subject which the test sample is derived from is mammal, preferably primate, more preferably human.

13. The method according to any one of claims 9-12, wherein the disease related to NT-proBNP is a cardiac disease;
further, the disease related to NT-proBNP is selected from heart failure, cardiac insufficiency, cardiogenic dyspnea, pulmonary dyspnea, acute coronary syndrome, or a combination thereof;
further, the heart failure is cardiogenic heart failure or non-cardiac heart failure.

14. A kit, comprising the binding protein as defined in any one of claims 1-3.

## Patentansprüche

1. Isoliertes Bindungsprotein, umfassend eine N-terminale Pro-Brain-Natriuretisches-Peptid-Antigen-Bindungsdomäne, wobei die Antigenbindungsdomäne sechs komplementaritätsbestimmende Regionen (CDR) der folgenden Aminosäuresequenzen umfasst;
die komplementaritätsbestimmende Region CDR-VH1 G-X1-S-X2-T-T-Y-Y-X3-D ist, wobei
X1 F ist, X2 I, V oder L ist, und X3 I, V oder L ist;
die komplementaritätsbestimmende Region CDR-VH2 M-T-K-D-X1-N-A-V-H-X2-P-T-X3-R-S ist, wobei
X1 G ist, X2 Q oder N ist, und X3 I, V oder L ist;
die komplementaritätsbestimmende Region CDR-VH3 V-X1-G-X2-I-D-X3-G ist, wobei
X1 R ist, X2 I, V oder L ist, und X3 F oder W ist;
die komplementaritätsbestimmende Region CDR-VL1 G-S-S-D-X1-V-G-X2-G-D-Y-X3-N ist, wobei
X1 Q oder N ist, X2 F ist und X3 I, V oder L ist;
die komplementaritätsbestimmende Region CDR-VL2 I-F-X1-A-X2-S-R-X3-R-G ist, wobei
X1 G ist, X2 T, Y oder S ist, und X3 I, V oder L ist;
die komplementaritätsbestimmende Region CDR-VL3 G-S-X1-N-S-R-X2-Y-V-X3-G ist, wobei
X1 P, A oder G ist, X2 GG oder N ist, und X3 F ist; die sechs komplementaritätsbestimmenden Regionen eine der folgenden Mutationskombinationen aufweisen:
| Stelle | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| Mutationskombination 1 | I/I | Q/I | I/W | O/V | T/I | P/GG |
| Mutationskombination 2 | I/L | N/I | I/F | Q/L | T/L | P/N |
| Mutationskombination 3 | I/V | Q/L | L/W | Q/I | T/V | A/GG |
| Mutationskombination 4 | L/I | N/L | L/F | N/V | Y/I | A/N |
| Mutationskombination 5 | L/L | Q/V | V/W | N/L | Y/L | G/GG |
| Mutationskombination 6 | L/V | N/V | V/F | N/I | Y/V | G/N |
| Mutationskombination 7 | V/I | N/V | I/W | N/I | S/I | P/N |
| Mutationskombination 8 | V/L | Q/V | I/F | N/L | S/L | G/N |
| Mutationskombination 9 | V/V | N/L | L/W | N/V | S/V | G/GG |
| Mutationskombination 10 | I/I | Q/L | L/F | Q/I | S/I | A/GG |
| Mutationskombination 11 | I/L | N/I | V/W | Q/L | S/L | A/N |
| Mutationskombination 12 | I/V | Q/I | V/F | Q/V | S/V | P/GG |
| Mutationskombination 13 | L/I | Q/I | I/W | Q/V | Y/I | P/GG |
| Mutationskombination 14 | L/L | N/I | I/F | Q/L | Y/L | P/N |
| Mutationskombination 15 | L/V | Q/L | L/W | Q/I | Y/V | A/GG |
| Mutationskombination 16 | V/I | N/L | L/F | N/V | T/I | A/N |
| Mutationskombination 17 | V/L | Q/V | V/W | N/L | T/L | G/GG |
| Mutationskombination 18 | V/V | N/V | V/F | N/I | T/V | G/N |
| Mutationskombination 19 | I/I | N/V | I/W | N/I | T/I | P/N |
| Mutationskombination 20 | I/L | Q/V | I/F | N/L | T/L | G/N |
| Mutationskombination 21 | I/V | N/L | L/W | N/V | T/V | G/GG |
| Mutationskombination 22 | L/I | Q/L | L/F | Q/I | Y/I | A/GG |
| Mutationskombination 23 | L/L | N/I | V/W | Q/L | Y/L | A/N |
| Mutationskombination 24 | L/V | Q/I | V/F | O/V | Y/V | P/GG |
| Mutationskombination 25 | V/I | Q/I | I/W | Q/V | S/I | P/GG |
| Mutationskombination 26 | V/L | N/I | I/F | Q/L | S/L | P/N |
| Mutationskombination 27 | V/V | Q/L | L/W | Q/V | S/V | A/GG |
| Mutationskombination 28 | I/I | N/L | L/F | N/V | S/I | A/N |
| Mutationskombination 29 | I/L | Q/V | V/W | N/L | S/L | G/GG |
| Mutationskombination 30 | I/V | N/V | V/F | N/I | S/V | G/N |
| Mutationskombination 31 | L/I | N/V | I/W | N/I | T/I | P/N |
| Mutationskombination 32 | L/L | Q/V | I/F | N/L | T/L | G/N |
| Mutationskombination 33 | L/V | N/L | L/W | N/V | T/V | G/GG |
| Mutationskombination 34 | V/I | Q/L | L/F | Q/I | Y/I | A/GG |
| Mutationskombination 35 | V/L | N/I | V/W | Q/L | Y/L | A/N |
| Mutationskombination 36 | V/V | Q/I | V/F | O/V | Y/V | P/GG |
| Mutationskombination 37 | I/I | Q/I | I/W | Q/V | T/I | P/GG |
| Mutationskombination 38 | I/L | N/I | I/F | Q/L | T/L | A/N |
| Mutationskombination 39 | I/V | Q/L | L/W | Q/I | T/V | G/GG |
| Mutationskombination 40 | L/I | N/L | L/F | N/V | Y/I | G/N |
| Mutationskombination 41 | L/L | Q/V | V/W | N/L | Y/L | P/N |
| Mutationskombination 42 | V/I | N/V | I/W | N/I | Y/V | G/N |
| Mutationskombination 43 | V/I | N/V | I/W | N/I | S/I | G/GG |
| Mutationskombination 44 | V/L | Q/V | I/F | N/L | S/L | A/GG |
| Mutationskombination 45 | V/V | N/L | L/W | N/V | S/V | A/N |
| Mutationskombination 46 | I/I | Q/L | L/F | Q/I | S/I | P/GG |
| Mutationskombination 47 | I/L | N/I | V/W | Q/L | S/L | P/GG |
| Mutationskombination 48 | I/V | Q/I | V/F | Q/V | S/V | P/N |
| Mutationskombination 49 | L/I | Q/I | I/W | Q/V | T/I | P/GG |
| Mutationskombination 50 | L/L | N/I | I/F | Q/L | T/L | P/N |
| Mutationskombination 51 | L/V | Q/L | L/W | Q/I | T/V | A/GG |
| Mutationskombination 52 | V/I | N/L | L/F | N/V | Y/I | A/N |
| Mutationskombination 53 | V/L | Q/V | V/W | N/L | Y/L | G/GG |
| Mutationskombination 54 | V/V | N/V | V/F | N/I | Y/V | G/N |
das Bindungsprotein Gerüstregionen der leichten Kette FR-L1, FR-L2, FR-L3 und FR-L4 mit Sequenzen umfasst, die entsprechend in SEQ ID NO: 1-4 gezeigt sind, und, Gerüstregionen der schweren Kette FR-H1, FR-H2, FR-H3 und FR-H4 mit Sequenzen, die entsprechend in SEQ ID NO: 5-8 gezeigt sind.

2. Isoliertes Bindungsprotein, umfassend eine N-terminale Pro-Brain-Natriuretisches-Peptid-Antigen-Bindungsdomäne nach Anspruch 1, wobei das Bindungsprotein eines von F(ab')₂, Fab', Fab, Fv, scFv und einem bispezifischen Antikörper ist.

3. Isoliertes Bindungsprotein, umfassend eine N-terminale Pro-Brain-Natriuretisches-Peptid-Antigen-Bindungsdomäne nach einem der Ansprüche 1 bis 2, wobei das Bindungsprotein ferner eine Sequenz der konstanten Region eines Antikörpers umfasst;
vorzugsweise die Sequenz der konstanten Region eine Sequenz einer beliebigen konstanten Region ist, die aus IgG1, IgG2, IgG3, IgG4, IgA, IgM, IgE und IgD ausgewählt ist;
vorzugsweise die konstante Region von Spezies abgeleitet ist, bestehend aus Rind, Pferd, Schwein, Schaf, Ziege, Ratte, Maus, Hund, Katze, Kaninchen, Kamel, Esel, Hirsch, Nerz, Huhn, Ente, Gans oder Mensch;
vorzugsweise die konstante Region von dem Schaf abgeleitet ist;
eine Sequenz der konstanten Region der leichten Kette in SEQ ID NO: 9 gezeigt ist;
eine Sequenz der konstanten Region der schweren Kette in SEQ ID NO: 10 gezeigt ist;
vorzugsweise das Bindungsprotein durch einen Indikator markiert ist, der eine Signalintensität zeigt.

4. Isoliertes Nukleinsäuremolekül, wobei das Nukleinsäuremolekül DNA oder RNA ist, dass das Bindungsprotein nach einem der Ansprüche 1 bis 3 codiert.

5. Vektor, umfassend die Nukleinsäure nach Anspruch 4.

6. Wirtszelle, die mit dem Vektor nach Anspruch 5 transformiert ist.

7. Verfahren zum Produzieren des Bindungsproteins nach einem der Ansprüche 1 bis 3,
das Verfahren umfassend die Schritte:
Kultivieren der Wirtszelle nach Anspruch 6 in einem Kulturmedium und unter geeigneten Kulturbedingungen, Gewinnen des derart hergestellten Bindungsproteins aus dem Kulturmedium oder aus der kultivierten Wirtszelle.

8. Verwendung des Bindungsproteins nach einem der Ansprüche 1 bis 3 bei der in vitro/ex vivo Diagnose von Herzversagen und Bewertung einer Herzfunktion, und in vitro/ex vivo Diagnose einer Krankheit, die mit NT-proBNP in Zusammenhang steht, wobei die Krankheit, die mit NT-proBNP in Zusammenhang steht, aus Herzversagen, Herzinsuffizienz, kardiogener Dyspnoe, pulmonaler Dyspnoe, akutem Koronarsyndrom oder einer Kombination davon ausgewählt ist;
wobei das Herzversagen kardiogenes Herzversagen oder nicht-kardiales Herzversagen ist.

9. Verfahren zum Erkennen von NT-proBNP in einer Testprobe, das Verfahren umfassend:
a) Inkontaktbringen des NT-proBNP-Antigens in der Testprobe mit dem Bindungsprotein nach einem der Ansprüche 1 bis 3, um einen Immunkomplex unter Bedingungen auszubilden, die für ein Stattfinden einer Antikörper/Antigen-Bindungsreaktion ausreichend sind; und
b) Erkennen einer Anwesenheit des Immunkomplexes, der eine Anwesenheit des NT-proBNP in der Testprobe angibt;
vorzugsweise in Schritt a) der Immunkomplex ferner einen zweiten Antikörper umfasst, der an das Bindungsprotein bindet;
vorzugsweise in Schritt a) der Immunkomplex ferner einen zweiten Antikörper umfasst, der an das NT-proBNP bindet;
vorzugsweise das Vorhandensein des Immunkomplexes das Vorhandensein einer Krankheit im Zusammenhang mit NT-proBNP angibt oder den Grad der Herzfunktion angibt.

10. Verfahren nach Anspruch 9, wobei das Verfahren auf Fluoreszenzimmunoassay, Chemilumineszenz, kolloidalem Gold-Immunoassay, Radioimmunoassay und/oder enzymgekoppeltem Immunoassay basiert.

11. Verfahren nach Anspruch 9 oder 10, wobei die Testprobe aus mindestens einem von Vollblut, peripherem Blut, Serum, Plasma oder Myokardgewebe ausgewählt ist.

12. Verfahren nach einem der Ansprüche 9 bis 11, wobei ein Subjekt, von dem die Testprobe stammt, ein Säugetier, vorzugsweise ein Primat, mehr bevorzugt ein Mensch, ist.

13. Verfahren nach einem der Ansprüche 9 bis 12, wobei die Krankheit, die mit NT-proBNP in Zusammenhang steht, eine Herzkrankheit ist;
ferner die Krankheit, die mit NT-proBNP in Zusammenhang steht, aus Herzversagen, Herzinsuffizienz, kardiogener Dyspnoe, pulmonaler Dyspnoe, akutem Koronarsyndrom oder einer Kombination davon ausgewählt ist;
ferner das Herzversagen ein kardiogenes Herzversagen oder ein nicht-kardiales Herzversagen ist.

14. Kit, umfassend das Bindungsprotein wie in einem der Ansprüche 1 bis 3 definiert.

## Revendications

1. Protéine de liaison isolée comprenant un domaine de liaison à l'antigène du peptide natriurétique pro-cérébral N-terminal, dans laquelle le domaine de liaison à l'antigène comprend six régions de détermination de complémentarité (CDR) des séquences d'acides aminés suivantes ;
la région de détermination de complémentarité CDR-VH1 est G-X1-S-X2-T-T-Y-Y-X3-D, dans laquelle
X1 est F, X2 est I, V ou L, et X3 est I, V ou L ;
la région de détermination de complémentarité CDR-VH2 est M-T-K-D-X1 -N-A-V-H-X2-P-T-X3-R-S, dans laquelle
X1 est G, X2 est Q ou N, et X3 est I, V ou L ;
la région de détermination de complémentarité CDR-VH3 est V-X1-G-X2-I-D-X3-G, dans laquelle
X1 est R, X2 est I, V ou L, et X3 est F ou W ;
la région de détermination de complémentarité CDR-VL1 est G-S-S-D-X1-V-G-X2-G-D-Y-X3-N, dans laquelle
X1 est Q ou N, X2 est F, et X3 est I, V ou L ;
la région de détermination de complémentarité CDR-VL2 est I-F-X1-A-X2-S-R-X3-R-G, dans laquelle
X1 est G, X2 est T, Y ou S, et X3 est I, V ou L ;
la région de détermination de complémentarité CDR-VL3 est G-S-X1-N-S-R-X2-Y-V-X3-G, dans laquelle
X1 est P, A ou G, X2 est GG ou N, et X3 est F ; les six régions de détermination de complémentarité ont l'une quelconque parmi les combinaisons de mutations suivantes :
| Site | CDR-VH1 X2/X3 | CDR-VH2 X2/X3 | CDR-VH3 X2/X3 | CDR-VL1 X1/X3 | CDR-VL2 X2/X3 | CDR-VL3 X1/X2 |
|---|---|---|---|---|---|---|
| Combinaison de mutations 1 | I/I | Q/I | I/W | Q/V | T/I | P/GG |
| Combinaison de mutations 2 | I/L | N/I | I/F | Q/L | T/L | P/N |
| Combinaison de mutations 3 | I/V | Q/L | L/W | Q/I | T/V | A/GG |
| Combinaison de mutations 4 | L/I | N/L | L/F | N/V | Y/I | A/N |
| Combinaison de mutations 5 | L/L | Q/V | V/W | N/L | Y/L | G/GG |
| Combinaison de mutations 6 | L/V | N/V | V/F | N/I | Y/V | G/N |
| Combinaison de mutations 7 | V/I | N/V | I/W | N/I | S/I | P/N |
| Combinaison de mutations 8 | V/L | Q/V | I/F | N/L | S/L | G/N |
| Combinaison de mutations 9 | V/V | N/L | L/W | N/V | S/V | G/GG |
| Combinaison de mutations 10 | I/I | Q/L | L/F | Q/I | S/I | A/GG |
| Combinaison de mutations 11 | I/L | N/I | V/W | Q/L | S/L | A/N |
| Combinaison de mutations 12 | I/V | Q/I | V/F | Q/V | S/V | P/GG |
| Combinaison de mutations 13 | L/I | Q/I | I/W | Q/V | Y/I | P/GG |
| Combinaison de mutations 14 | L/L | N/I | I/F | Q/L | Y/L | P/N |
| Combinaison de mutations 15 | L/V | Q/L | L/W | Q/I | Y/V | A/GG |
| Combinaison de mutations 16 | V/I | N/L | L/F | N/V | T/I | A/N |
| Combinaison de mutations 17 | V/L | Q/V | V/W | N/L | T/L | G/GG |
| Combinaison de mutations 18 | V/V | N/V | V/F | N/I | T/V | G/N |
| Combinaison de mutations 19 | I/I | N/V | I/W | N/I | T/I | P/N |
| Combinaison de mutations 20 | I/L | Q/V | I/F | N/L | T/L | G/N |
| Combinaison de mutations 21 | I/V | N/L | L/W | N/V | T/V | G/GG |
| Combinaison de mutations 22 | L/I | Q/L | L/F | Q/I | Y/I | A/GG |
| Combinaison de mutations 23 | L/L | N/I | V/W | Q/L | Y/L | A/N |
| Combinaison de mutations 24 | L/V | Q/I | V/F | Q/V | Y/V | P/GG |
| Combinaison de mutations 25 | V/I | Q/I | I/W | Q/V | S/I | P/GG |
| Combinaison de mutations 26 | V/L | N/I | I/F | Q/L | S/L | P/N |
| Combinaison de mutations 27 | V/V | Q/L | L/W | Q/V | S/V | A/GG |
| Combinaison de mutations 28 | I/I | N/L | L/F | N/V | S/I | A/N |
| Combinaison de mutations 29 | I/L | Q/V | V/W | N/L | S/L | G/GG |
| Combinaison de mutations 30 | I/V | N/V | V/F | N/I | S/V | G/N |
| Combinaison de mutations 31 | L/I | N/V | I/W | N/I | T/I | P/N |
| Combinaison de mutations 32 | L/L | Q/V | I/F | N/L | T/L | G/N |
| Combinaison de mutations 33 | L/V | N/L | L/W | N/V | T/V | G/GG |
| Combinaison de mutations 34 | V/I | Q/L | L/F | Q/I | Y/I | A/GG |
| Combinaison de mutations 35 | V/L | N/I | V/W | Q/L | Y/L | A/N |
| Combinaison de mutations 36 | V/V | Q/I | V/F | Q/V | Y/V | P/GG |
| Combinaison de mutations 37 | I/I | Q/I | I/W | Q/V | T/I | P/GG |
| Combinaison de mutations 38 | I/L | N/I | I/F | Q/L | T/L | A/N |
| Combinaison de mutations 39 | I/V | Q/L | L/W | Q/I | T/V | G/GG |
| Combinaison de mutations 40 | L/I | N/L | L/F | N/V | Y/I | G/N |
| Combinaison de mutations 41 | L/L | Q/V | V/W | N/L | Y/L | P/N |
| Combinaison de mutations 42 | V/I | N/V | I/W | N/I | Y/V | G/N |
| Combinaison de mutations 43 | V/I | N/V | I/W | N/I | S/I | G/GG |
| Combinaison de mutations 44 | V/L | Q/V | I/F | N/L | S/L | A/GG |
| Combinaison de mutations 45 | V/V | N/L | L/W | N/V | S/V | A/N |
| Combinaison de mutations 46 | I/I | Q/L | L/F | Q/I | S/I | P/GG |
| Combinaison de mutations 47 | I/L | N/I | V/W | Q/L | S/L | P/GG |
| Combinaison de mutations 48 | I/V | Q/I | V/F | Q/V | S/V | P/N |
| Combinaison de mutations 49 | L/I | Q/I | I/W | Q/V | T/I | P/GG |
| Combinaison de mutations 50 | L/L | N/I | I/F | Q/L | T/L | P/N |
| Combinaison de mutations 51 | L/V | Q/L | L/W | Q/I | T/V | A/GG |
| Combinaison de mutations 52 | V/I | N/L | L/F | N/V | Y/I | A/N |
| Combinaison de mutations 53 | V/L | Q/V | V/W | N/L | Y/L | G/GG |
| Combinaison de mutations 54 | V/V | N/V | V/F | N/I | Y/V | G/N |
la protéine de liaison comprend des régions de charpente de chaîne légère FR-L1, FR-L2, FR-L3 et FR-L4 avec des séquences montrées de manière correspondante dans SEQ ID NO: 1 à 4, et, des régions de charpente de chaîne lourde FR-H1, FR-H2, FR-H3 et FR-H4 avec des séquences montrées de manière correspondante dans SEQ ID NO: 5 à 8.

2. Protéine de liaison isolée comprenant un domaine de liaison à l'antigène du peptide natriurétique pro-cérébral N-terminal selon la revendication 1, dans laquelle la protéine de liaison est l'un parmi F(ab')₂, Fab', Fab, Fv, scFv, et un anticorps bispécifique.

3. Protéine de liaison isolée comprenant un domaine de liaison à l'antigène du peptide natriurétique pro-cérébral N-terminal selon l'une quelconque des revendications 1 à 2, dans laquelle la protéine de liaison comprend en outre une séquence de région constante d'anticorps ;
de préférence, la séquence de région constante est une séquence de l'une quelconque région constante choisie parmi IgG1, IgG2, IgG3, IgG4, IgA, IgM, IgE et IgD ;
de préférence, la région constante est dérivée d'espèces constituées de bovin, cheval, porc, mouton, chèvre, rat, souris, chien, chat, lapin, chameau, âne, cerf, vison, poulet, canard, oie ou humain ;
de préférence, la région constante est dérivée du mouton ;
une séquence de région constante de chaîne légère est montrée dans SEQ ID NO: 9 ;
une séquence de région constante de chaîne lourde est montrée dans SEQ ID NO: 10 ;
de préférence la protéine de liaison est marquée par un indicateur qui montre une intensité de signal.

4. Molécule d'acide nucléique isolée, la molécule d'acide nucléique étant de l'ADN ou de l'ARN, qui code pour la protéine de liaison selon l'une quelconque des revendications 1 à 3.

5. Vecteur, comprenant l'acide nucléique selon la revendication 4.

6. Cellule hôte transformée avec le vecteur selon la revendication 5.

7. Procédé permettant de produire la protéine de liaison selon l'une quelconque des revendications 1 à 3, le procédé comprenant les étapes consistant à :
cultiver la cellule hôte selon la revendication 6 dans un milieu de culture et dans des conditions de culture appropriées, récupérer une telle protéine de liaison produite à partir du milieu de culture ou de la cellule hôte cultivée.

8. Utilisation de la protéine de liaison selon l'une quelconque des revendications 1 à 3 dans le diagnostic in vitro/ex vivo d'une défaillance cardiaque et l'évaluation d'une fonction cardiaque, et le diagnostic in vitro/ex vivo d'une maladie liée au NT-proBNP, dans laquelle la maladie liée au NT-proBNP est choisie parmi défaillance cardiaque, insuffisance cardiaque, dyspnée cardiogénique, dyspnée pulmonaire, syndrome coronarien aigu, ou une combinaison de ceux-ci ;
dans laquelle, la défaillance cardiaque est une défaillance cardiaque cardiogénique ou une défaillance cardiaque non cardiogénique.

9. Procédé permettant de détecter NT-proBNP dans un échantillon de test, le procédé comprenant :
a) la mise en contact de l'antigène NT-proBNP dans l'échantillon de test avec la protéine de liaison selon l'une quelconque des revendications 1 à 3 pour former un complexe immun dans des conditions suffisantes pour permettre une réaction de liaison anticorps/antigène ; et
b) la détection d'une présence du complexe immun, qui indique une présence du NT-proBNP dans l'échantillon de test ;
de préférence, à l'étape a), le complexe immun comprend en outre un second anticorps qui se lie à la protéine de liaison ;
de préférence, à l'étape a), le complexe immun comprend en outre un second anticorps qui se lie au NT-proBNP ;
de préférence, la présence du complexe immun indique la présence d'une maladie liée au NT-proBNP ou indique le niveau de la fonction cardiaque.

10. Procédé selon la revendication 9, dans lequel le procédé est basé sur un dosage immunologique par fluorescence, une chimiluminescence, un dosage immunologique à l'or colloïdal, un dosage radio-immunologique et/ou un dosage immunoenzymatique.

11. Procédé selon la revendication 9 ou 10, dans lequel l'échantillon de test est choisi parmi au moins l'un parmi sang total, sang périphérique, sérum, plasma ou tissu myocardique.

12. Procédé selon l'une quelconque des revendications 9 à 11, dans lequel un sujet duquel l'échantillon de test est dérivé est un mammifère, de préférence un primate, plus préférablement un humain.

13. Procédé selon l'une quelconque des revendications 9 à 12, dans lequel la maladie liée au NT-proBNP est une maladie cardiaque ;
en outre, la maladie liée au NT-proBNP est choisie parmi défaillance cardiaque, insuffisance cardiaque, dyspnée cardiogénique, dyspnée pulmonaire, syndrome coronarien aigu, ou une combinaison de ceux-ci ;
en outre, la défaillance cardiaque est une défaillance cardiaque cardiogénique ou une défaillance cardiaque non cardiogénique.

14. Trousse, comprenant la protéine de liaison selon l'une quelconque des revendications 1 à 3.
